# EUROPEAN PATENT SPECIFICATION

(11) **EP 4 210 667 B1**
(45) Date of publication and mention of the grant of the patent: **15.01.2025**
(21) Application number: 21766529.8
(22) Date of filing: 08.09.2021
(51) Int. Cl.: A61K 9/00, A61K 9/06, A61K 47/14, A61K 47/26, A61K 47/34, A61K 47/44

(54) **DERMAL COMPOSITIONS REPLICATING THE VERNIX CASEOSA**
DERMALE ZUSAMMENSETZUNGEN ZUR REPLIKATION DES VERNIX CASEIA
COMPOSITIONS DERMIQUES RÉPRODUISANT LE VERNIX CASEOSA

(30) Priority: 08.09.2020 IT 202000021223
(43) Date of publication of application: 19.07.2023
(73) Proprietor: APR APPLIED PHARMA RESEARCH S.A., 6828 Balerna (CH)
(72) Inventor: REINER, Giorgio, 22100 Como (IT); ZOCCA, Barbara, 22020 Faloppio (Como) (IT)
(74) Representative: Vossius & Partner Patentanwälte Rechtsanwälte mbB
(86) International application number: PCT/IB2021/058174
(87) International publication number: WO 2022/053952

(56) References cited:
- WO-A1-2020/086755
- TANSIRIKONGKOL ANYARPORN ET AL: "Water-handling properties of vernix caseosa and a synthetic analogue", JOURNAL OF COSMETIC SCIENCE, SOCIETY OF COSMETIC CHEMISTS, NEW YORK, NY, US, vol. 58, no. 6, 1 November 2007 (2007-11-01), pages 651 - 662, XP009121718, ISSN: 1525-7886

## Description

### FIELD OF THE INVENTION

The present invention relates to synergistic combination of ingredients intended for the preparation of products for the care and protection of skin and mucosal membranes, particular in the treatment of skin and mucosal diseases such as atopic dermatitis and wound healing, but not limited to these.

### BACKGROUND OF THE INVENTION

The human skin plays an important role in the maintenance of a living body through skin barrier function including biological protection against external stimuli such as physical impacts, temperature, exposure to ultraviolet rays or chemicals, or infection, and the moisturizing function that prevents water loss from inside a living body but also prevent excessive water absorption thanks to its water resistance capacity.

The human skin barrier has two primary physiological and physical barrier functions as an "exit" and an "entrance" barrier. As an exit barrier, the skin barrier maintains skin moisturization by preventing vital water of the skin from exiting the body (i.e., evaporation of water into the air). Conversely, as an entrance barrier, the skin barrier minimizes infiltration by microbial and other foreign contaminants by preventing entrance of potential toxins, pollutants, infectants, and allergens, into the body. Skin also plays a key metabolic role in vitamin D synthesis and biotransformation of some chemicals.

In terms of chemical composition the skin is about 70% water, 25% protein and 2% lipids. The skin consists of three main layers: epidermis, dermis and subcutaneous tissue. The epidermis is made up of 5 layers that correspond to the same cells at different times of their life cycle, from the inner to outer they are : basal or germinal, spinous, granular, clear or translucent, cornified or horny layer or stratum corneum, this last is the target of dermatological treatment.

Skin water content is managed in two ways : as active transport by sweating (perspiration sensibilis) and as passive diffusion through the horny layer (perspiration insensibilis also TEWL Trans Epidermal Water Loss). The diffusion barrier is situated entirely within the horny layer.

Perspiration insensibilis or TEWL is a passive phenomenon due to the water vapor pressure gradients on both sides of the layer, as water concentration in the epidermidis, being in contact with the dermis is higher than the water concentration at skin surface which is in contact with drier surrounding. Thus if the relative humidity of the surrounding air is 100% the TEWL will decrease to almost zero and inversely if the relative humidity of the surrounding air is 0% the TEWL will be maximal.

The skin has another way to regulate its hydric capital an emulsion of water and water soluble substances and lipids known as the hydro-lipidic film, a protective film that covers the entire surface of the skin. It is composed mainly of sweat, sebum and water which acts as a barrier to protect the skin from external factors and prevent the escape of moisture from the skin.

If the horny layer barrier function is impaired, increased water loss, decrease of hydration, itching, resorption of toxic or allergenic substances or attack by microorganisms may result, leading to toxic or allergic or inflammatory skin reaction. The health and integrity of skin may also be compromised by wounds, abrasions, ulcers, burns, infections, irritations, premature birth and other conditions for which normal skin production and repair processes may be inadequate. Skin problems such as decubitus ulcers or irritations from diaper rash may progress to more severe conditions if left untreated.

The maintenance of horny layer barrier function is the target of dermatological treatment. Tansirikongkol et al (J. Cosmet. Sci., 58, 651-662, 2007) discloses a synthetic analogue of the vernix caseosa.

### SUMMARY OF THE INVENTION

The invention provides a composition of ingredients according to the claims able to obtain a breathable non-occlusive skin or mucosal protective effect. The breathable non occlusive protective barrier is intended to provide a skin or mucosa protective barrier with a water vapour transmission rate and occlusivity similar to that of the vernix caseosa.

The key ingredients are hydrogenated polydecene, trihydroxystearin, bis-octyldodecyl dimer dilinoleate/propanediol copolymer, betaine and xylitol. This technology has a dual action: it works as a breathable barrier protecting the skin (or mucosa) from the potential entry of allergens, pollutants, and external toxins while at the same time allowing the skin to perform its physiological transpiration action. This balanced dual action avoids the excessive water loss typical of impaired skin as well as the excessive presence of water that might lead to maceration. Thanks to the application of a breathable non occlusive protective barrier, the products mimic the skin/mucosa barrier's function.

### DETAILED DESCRIPTION

The composition according to the present invention is a synergistic combination of ingredients according to the claims intended for the formulation of products for the care and protection of the skin or mucosa able to function as a breathable non occlusive skin or mucosa protective barrier. The breathable non occlusive protective barrier is intended as a skin or mucosa protective barrier with a water vapour transmission rate and occlusivity similar to that of vernix caseosa.

### VERNIX CASEOSA

The vernix caseosa ("vernix") is a naturally occurring skin protectant that progressively covers the skin of the developing fetus completely surrounded by amniotic fluid during the last trimester of pregnancy. Vernix caseosa consists mainly of water (80%), lipids (10%) and proteins (10%) and provides a multi-component defense system based on polypeptides, lipids, and their interactions. Vernix caseosa is a naturally occurring emulsion covering the skin surface of the fetus in the last trimester of pregnancy, produced in part by fetal sebaceous glands. The production of vernix coincides with the presence of the terminally differentiated epidermis as seen by the formation of the fetal stratum corneum.

Once a baby is delivered, the vernix caseosa has two important external functions: it maintain a newborns' body temperature by regulating the newborn's transepidermal water loss (TEWL); when the relative humidity is lowered, as happens during delivery, water is released from the vernix to the stratum corneum, ensuring that the imperfect stratum corneum of the newborn will have sufficient water for all its enzymes to function properly. For this reason, in the present invention, vernix and its unique water-holding capability has been considered a gold standard reference in the selection of the synergistic combination of ingredients of this invention.

### BALANCED WATER-HOLDING CAPABILITY

The importance of a balanced water-holding capability is also linked to knowledge that an excessive increase in hydration in the stratum corneum caused by the application of occlusive substances that limit the perspiration insensibilis is not conducive to the well-being and protection of the skin but on the contrary undergoes alterations that originate from the morphological and functional degree of the barrier. Excessive imbibition, also named as maceration, involves a disarrangement of the skin barrier as a whole. Maceration leads to several skin disorders, to name but a few:
the swelling of corneocytes that leads to the separation of the lipidic lamellar, that reduces the cohesion and mechanical resistance of the stratum corneum, the inhibition of filaggrin breakdown, the shift to basic pH values in the skin that jeopardize enzymatic processes necessary for the construction of the barrier and reduce the skin antimicrobial properties.

The functional recovery of the barrier is normally achieved if the product applied to the skin is permeable to water. In contrast to occlusion membranes, vapor-permeable membranes allow barrier function to recover normally.

### KEY INGREDIENTS

The ingredients of the present invention belong to the following chemical groups:
- hydrogenated oligomers of alpha olefin, polyalkanes, isoparaffins with carbon chain length of 20 to 60, such us hydrogenated polyisobutene, hydrogenated polybutene, hydrogenated didecene, hydrogenated polydecene, C13-15 Alkanes to C21-28 Alkane
- triesters of glycerin and branched or linear saturated fatty acids, such us glyceryl tristearate, glyceryl tripalmitate, glyceryl benehate, triisostearin, trihydroxystearin, glyceryl triacetyl hydroxystearate, glyceryl tribehenate/isostearte/eicosandioate, glyceryl triacetyl rosinate
- Dilinoleate/Propanediol backbone Bis-Octyldodecyl Dimer Dilinoleate/Propanediol Copolymer (Bis Octyldodecyl Dimer Dilinoleate/Propanediol Copolymer)
- a small amino acid which is alanine, valine, leucine, isoleucine, glycine, proline and mixtures thereof, or the small amino acid is trimethylglycine
- Sugar alcohols such as glycerol, erythritol, xylitol, mannitol, sorbitol, inositol, isomalt, maltitol, lactitol

The preferred ingredients of the present invention are hydrogenated polydecene, trihydroxystearin, Bis-Octyldodecyl Dimer Dilinoleate/Propanediol Copolymer, trimethylglycine (betaine), and xylitol. The technical features of these ingredients are given below.

### HYDROGENATED POLYDECENE

Hydrogenated polydecene is a colourless, odourless, viscous, fully apolar oily liquid, completely insoluble in water and with low volatility. Hydrogenated Polydecene is an ingredient known also to the US food industry that use it as glazing agent, to give food a shiny appearance. It also provides a protective coating, used, for example, on fruits and certain sugar confectionery, such as fruit gums and jellies.

Chemically is a fully saturated poly α olefin, a long branched hydrocarbon chain polymer, with an average molecular around 500 Dalton. According to 'the 500 Dalton Rule' a molecule with a weight 500 Dalton or greater is too large and bulky to be able to go by the horny layer, so this molecule shows a very low dermal bioavailability. Hydrogenated polydecene is chemically highly stable, its use in the context of dermocosmetic skin treatment, is due to its absolute inertia and neutrality for the skin. On the other side, for their rounded molecular shape its molecules have extended lubricant properties, so reducing the friction between the skin and clothes, developing a pronounced emollient effect.

### Bis-Octyldodecyl Dimer Dilinoleate/Propanediol Copolymer

Bis Octyldodecyl Dimer Dilinoleate/Propanediol Copolymer has been selected for its peculiar effect on oil phases. Bis Octyldodecyl Dimer Dilinoleate/Propanediol Copolymer is a 'high-engineering' polymer, more properly a Multi-Domain Polymer, thus a polymer that incorporates in the same structure chemical groups with opposite chemical-physical features, each one covalently bonded onto the same polymer backbone. This gives the polymer unique and unexpected properties. In the case of Bis Octyldodecyl Dimer Dilinoleate/Propanediol Copolymer, the Dimer Dilinoleate/Propanediol backbone brings two different alkyl groups (Cs) and dodecyl (C₁₂). Its structure shows the unexpected property of lowering the surface tension of oils, a very surprising and unexpected result as, typically, oil soluble materials do not decrease the surface tension of the oil where they are dissolved. Thanks to this property, Bis Octyldodecyl Dimer Dilinoleate/Propanediol Copolymer is able to reduce the surface tension of hydrogenated polydecene and helps its spreading over the skin.

The surface tension reduction of Bis Octyldodecyl Dimer Dilinoleate/Propanediol Copolymer is able to change the wetting properties of hydrogenated polydecene. Bis Octyldodecyl Dimer Dilinoleate/Propanediol.

### TRIHYDROXYSTEARINE

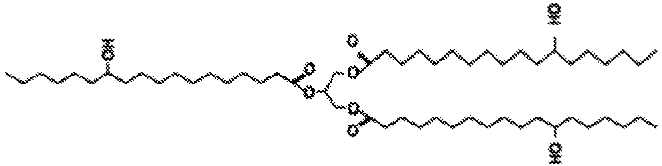

Trihydroxystearine is the triester of glycerin and hydroxystearic acid, it is composed of 57 atoms of carbon, 110 atoms of hydrogen and 9 atoms of oxygen, so its molecular weight is more than 900 Dalton. It acts as a gellant, viscosity increaser, rheological controller of oily phase; on skin it acts as a perspiration controller and water repellent by plugging the gap between skin cells (corneocytes).

### BETAINE

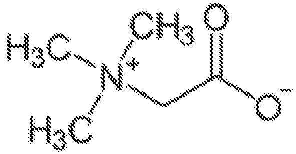

Betaine or Trimethyl Glycine (TMG) is derived from the sugar beet (Beta vulgaris); its commercial form is obtained from the process of making sugar out of sugar beets, betaine tastes sweet. It is a small trimethylated aminoacid existing in a zwitterionic form at neutral pH. Betaine is a cellular osmoprotector, secreted by marine microorganisms to help them resist osmotic stress. Differential Scanning Calorimetry shows a water-betaine eutectic point at -22°C at 15% concentration; a 50% aqueous solution freezes at -32°C. Wheat plants increase their resistance to biotrophic fungi by a pre-treatment with betaine; betaine improves the drought and chill resistance of growing plants.

Because of its structure, betaine can easily form hydrogen bonds with water and other polar molecules conferring it very water carrier properties. In aqueous solutions betaine forms strong hydrogen bonds (8 to 9 kcal/mole), thus changing the water activity. The carboxylic group (COO-) attracts the hydrogen atoms of the surrounding water. Additional water molecules are spatially oriented around betaine under two opposite forces: attraction, by the positive charge that is present on the nitrogen atom (N), and repulsion, by the methyl groups (CH3) that are attached to the same atom. The Huggins constant (k'), which is generally interpreted as an interaction factor accounting for the easy exchange of water molecules between the hydration sphere of betaine and the bulk water, is high (k' = 1.156). Also the negative hydration number (h = -0.797) indicates that the statistical average number of water molecules remaining constantly around the betaine molecule is very low. In other words, both values suggest that one water molecule, when attracted by a betaine molecule, is very rapidly substituted by another water molecule. The mobility of the three methyl groups around the C-N axis and their preferential but mobile arrangements due to the steric hindrance, explain why water molecules are quickly released from the hydration sphere near betaine to the bulk of the solution. Betaine is therefore a true water carrier that releases it easily to the surrounding environment when require. Furthermore, betaine does not immobilize water molecules as many humectant polyols (like glycerol) do. This allows the water for the living cells to be completely available.

### XYLITOL

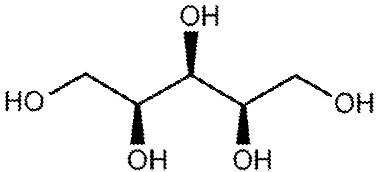

Xylitol is a polyalcohol, which is well known because of its use as sugar replacement in candy and food in general. Xylitol occurs freely in fruits and other plant parts; it is also present in human metabolism as a normal metabolic intermediate. Chemically it is a sugar alcohol and a polyol. Owing to its 8 hydroxyl group (OH) it is highly polar molecular, with a great affinity to water. Like betaine and with betaine too, it creates ionic bonds with water thus helping to increase the water content in the stratum corneum as it locks moisture onto the surface of the skin.

Xylitol could exert also the function of help in staphylococcus aureus colonization reduction; being recognized that colonization by Staphylococcus aureus on the skin is one of the factors that can worsen atopic dermatitis. Xylitol has not properly an antimicrobial activity on Staphylococcus aureus. It mainly has anti-adherent property inhibits the formation of the substance that surround the cell membrane (glycocalyx), the adjuvant action of xylitol is important since glycocalyx is the primary structure responsible for bacterial drug resistance.

### IN VITRO TEST VERSUS VERNIX CASEOSA

An in vitro test developed by Johann Wiechers has been the screening method selected to develop dermatological preparation with occlusivity and a water vapour transmission rate close to vernix caseosa. The test foresees the deposition of the test substances over a substrate that effectively mimics the surface properties of human skin (IMS Vitro-skin membrane), and a precisely weighed quantity of water beneath this membrane.

The kit is weighed and then maintained at 34°C and 54% relative humidity to mimic skin physiological conditions for 10 hours, then weighed to evaluate the quantity of water lost (i.e. passed through the tested substance) or held inside by the tested substance. The test results are expressed in two ways: as OCCLUSIVITY FACTOR (F) and as WATER VAPOUR TRASMISSION RATE (WVTR). The occlusivity factor (F) is a measurement of the water retention capacity of the tested substance. The WVTR is a measurement of the water loss through the substance, or the test substance barrier's breathability.

Occlusivity factor (F) is calculated as: [(A - B) / A] x 100, where A is the amount of water passed through the untreated membrane, and B is the amount of water passed through the membrane treated with test substance. A and B are calculated as the difference between a sample's weight after storage time (T1) and before storage time (T0).

WVTR is calculated as: water loss (g)/surface (m²)/time (h).

### PROTOTYPE DEVELOPMENT

The aim of this development was to obtain topical formulations with an occlusivity factor and a water vapor transmission rate statistically similar to vernix caseosa in terms of occlusivity factor and water vapor transmission rate.

The first set of formulas tested versus vernix caseosa was identified as Reference 2952 Prototype 19 and Reference 2953 Prototype 20, both of which are not within the scope of the claims. Quali-quantitative formulas of these prototypes is the following TABLE 1:

**TABLE 1**

| Ingredients (INCI Name) | Ref 2952-19 | Ref 2953-20 |
|---|---|---|
| AQUA | to 100% | to 100% |
| HYDROGENATED POLYDECENE | 15 | 13 |
| POLYGLYCERYL-2 | - | 4 |
| DIPOLYHYDROXYSTEARATE | | |
| ISOSTEARYL ISOSTEARATE | - | 2.5 |
| POLYGLYCERYL-3 DIISOSTEARATE | 3 | 2 |
| PEG-30 DIPOLYHYDROXYSTEARATE | 2 | - |
| BETAINE | 2 | 2 |
| GLYCERYL BEHENATE | 1.5 | 2 |
| GLYCERYL LAURATE | 1 | 1 |
| MAGNESIUM STEARATE | 0.5 | 1 |
| MAGNESIUM SULPHATE | 0.5 | 1 |
| SODIUM CHLORIDE | 0.5 | 0.5 |
| GLYCERYL UNDECILENATE | 0.5 | 0.5 |
| POTASSIUM SORBATE | 0.25 | 0.25 |
| DISODIUM EDTA | 0.2 | 0.2 |
| PHENYLPROPANOL | 0.2 | 0.2 |
| LACTIC ACID | 0.275 | 0.620 |
| TOCOPHEROL | 0.05 | 0.05 |

### RESULTS OF IN VITRO TEST VERSUS VERNIX CASEOSA

Surprising results obtained in the in vitro test versus vernix caseosa and petrolatum are reported in TABLE 2:

**TABLE 2**

| **Tested Substances** | **Occlusion Factor (F)** | **Difference vs VC** | **WVTR** | **Difference vs VC** |
|---|---|---|---|---|
| **Vernix Caseosa (VC)** | 13.1 | - | 48.25 | - |
| Petrolatum | 47.4 | + 34.4 | 29.21 | + 19.04 |
| Ref 2952-19 | 9.9 | - 3.2 | 50.02 | + 1.77 |
| Ref 2953-20 | 8.6 | - 4.5 | 50.73 | + 2.48 |

Ref 2952-19 and the Ref 2953-20 (both not within the scope of the claims) did not show any statistically significant difference in terms of occlusivity and water vapour transmission rate when compared to vernix caseosa. However, they did show a statistically significant difference in term of occlusivity and water vapour transmission rate when compared to petrolatum.

### PROTOTYPE ADJUSTMENT

Even though the results obtained on Ref 2952-19 and the Ref 2953-20 (both not within the scope of the claims) were within an acceptable range, and their occlusivity factors and water vapour transmission rates were comparable to vernix caseosa in a statistically significant way, we adjusted the formula in order to narrow the difference with vernix caseosa further in term of occlusivity and water vapour transmission rate. Formula adjustment led from Ref 2952-19 (not within the scope of the claims) to Ref 2952-20 and from Ref 2953-20 (not within the scope of the claims) to Ref 2953-29. TABLE 3 and TABLE 4 compare the formulas of Ref 2952 and Ref 2953, showing the formula adjustment performed.

**TABLE 3**

| Ingredients (INCI Name) | Ref 2952-19 | Ref 2952-20 |
|---|---|---|
| AQUA | to 100% | to 100% |
| HYDROGENATED POLYDECENE | 15 | 15 |
| POLYGLYCERYL-3 DIISOSTEARATE | 3 | 3 |
| PEG-30 DIPOLYHYDROXYSTEARATE | 2 | 2 |
| BIS-OCTYLDODECYL DIMER | - | 3 |
| DILINOLEATE/PROPANEDIOL COPOLYMER | | |
| BETAINE | 2 | 2 |
| XYLITOL | - | 2 |
| GLYCERYL BEHENATE | 1.5 | - |
| GLYCERYL LAURATE | 1 | - |
| TRIHYDROXYSTEARIN | - | 1 |
| MAGNESIUM STEARATE | 0.5 | 0.5 |
| MAGNESIUM SULPHATE | 0.5 | 0.7 |
| SODIUM CHLORIDE | 0.5 | - |
| GLYCERYL UNDECILENATE | 0.5 | - |
| HYDROXYACETOPHENONE | - | 0.3 |
| CHLORPHENESIN | - | 0.25 |
| POTASSIUM SORBATE | 0.25 | - |
| DISODIUM EDTA | 0.2 | 0.2 |
| PHENYLPROPANOL | 0.2 | - |
| LACTIC ACID | 0.275 | 0.125 |
| O-CYMEN-5-OL | - | 0.1 |
| TOCOPHEROL | 0.05 | - |

The differences among the formulas are: (i) insertion of Bis Octyldodecyl Dimer Dilinoleate/Propanediol Copolymer, (ii) replacement of the glycerin esters (glyceryl laurate, glyceryl behenate, glyceryl undecilenate) with hydroxystearic acid (trihydroxystearin), (iii) boosting the hydrophilic phase by insertion of betaine (trimethylglycine) and removal of sodium chloride, and (iv) modifications of the preservative system with no impact on occlusivity or WVTR.

**TABLE 4**

| Ingredients (INCI Name) | Ref2953-20 | Ref 2953-29 |
|---|---|---|
| AQUA | to 100% | to 100% |
| HYDROGENATED POLYDECENE | 13 | 12 |
| POLYGLYCERYL-2 DIPOLYHYDROXYSTEARATE | 4 | 4 |
| ISOSTEARYL ISOSTEARATE | 2.5 | 3 |
| POLYGLYCERYL-3 DIISOSTEARATE | 2 | 2 |
| BIS-OCTYLDODECYL DIMER | - | 3 |
| DILINOLEATE/PROPANEDIOL COPOLYMER | | |
| BETAINE | 2 | 2 |
| XYLITOL | - | 2 |
| GLYCERYL BEHENATE | 2 | - |
| GLYCERYL LAURATE | 1 | - |
| TRIHYDROXYSTEARIN | - | 1 |
| MAGNESIUM STEARATE | 1 | 1 |
| MAGNESIUM SULPHATE | 1 | 0.7 |
| SODIUM CHLORIDE | 0.5 | - |
| GLYCERYL UNDECILENATE | 0.5 | - |
| HYDROXYACETOPHENONE | - | 0.3 |
| CHLORPHENESIN | - | 0.25 |
| POTASSIUM SORBATE | 0.25 | - |
| DISODIUM EDTA | 0.2 | 0.2 |
| PHENYLPROPANOL | 0.2 | - |
| LACTIC ACID | 0.620 | 0.150 |
| O-CYMEN-5-OL | - | 0.1 |
| TOCOPHEROL | 0.05 | - |

The difference among the formulas are: (i) insertion of Bis Octyldodecyl Dimer Dilinoleate/Propanediol Copolymer , (ii) adjustment in ratio among hydrogenated polydecene and isosterayl isosterate, (iii) replacement of glycerin esters (glyceryl laurate, glyceryl behenate, glyceryl undecilenate) with hydroxystearic acid (trihydroxystearin), (iv) boosting the hydrophilic phase by insertion of betaine (trimethylglycine) and removal of sodium chloride, and (v) other modifications in the preservative system with no impact on occlusivity or WVTR.

### RESULTS OF IN VITRO TEST VS VERNIX CASEOSA ON ADJUSTED PROTOTYPES

Ref 2952-20 and Ref 2953-29 were tested in vitro versus vernix caseosa with the following results in TABLE 5:

**TABLE 5**

| **Tested Substances** | **Occlusion Factor (F)** | **Difference vs VC** | **WVTR** | **Difference vs VC** |
|---|---|---|---|---|
| **Vernix Caseosa** | 23.87 | - | 50.28 | - |
| Petrolatum | 75.65 | + 51.78 | 16.07 | - 34.21 |
| Ref 2952-20 | 22.47 | - 1.40 | 51.15 | + 0.88 |
| Ref 2953-29 | 24.94 | + 1.07 | 49.56 | -0.72 |

Surprisingly, the formula's adjustment reached the goal as shown in comparison TABLE 6:

**TABLE 6**

| | **Δ Occlusion Factor (F)** | **Δ WVTR** |
|---|---|---|
| **Ref 2952 - 19** | - 3.2 | + 1.77 |
| **Ref 2952 - 20** | - 1.4 | + 0.88 |
| **Ref 2953 - 20** | - 4.5 | + 2.48 |
| **Ref 2953** - **29** | +1.07 | -0.72 |

Based on these results, it can be concluded that there is a synergistic action among hydrogenated polycedene, trihydroxystearin, Bis Octyldodecyl Dimer Dilinoleate/Propanediol Copolymer, betaine, xylitol, able to obtain formulas with occlusion factor and water vapour transmission rate statistically significant close to vernix caseosa.

### OTHER PROTOTYPES

To confirm the synergistic action of hydrogenated polycedene, trihydroxystearin, Bis Octyldodecyl Dimer Dilinoleate/Propanediol Copolymer, betaine, and xylitol, in terms of occlusion factor and water vapour transmission rate, these ingredients were introduced into the compositions of TABLE 7 and TABLE 8:

**TABLE 7**

| Ingredients (INCI Name) | **2954-12** |
|---|---|
| AQUA | to 100% |
| HYDROGENATED POLYDECENE | 15 |
| POLYGLYCERYL-2 DIPOLYHYDROXYSTEARATE | 4 |
| BIS-OCTYLDODECYL DIMER | 3 |
| DILINOLEATE/PROPANEDIOL COPOLYMER | |
| POLYGLYCERYL-3 DIISOSTEARATE | 2 |
| BETAINE | 2 |
| XYLITOL | 2 |
| DEFENSII, PLUS^{$} | 1.5 |
| TRIHYDROXYSTEARIN | 1 |
| MAGNESIUM STEARATE | 1 |
| MAGNESIUM SULPHATE | 0.7 |
| HYDROXYACETOPHENONE | 0.3 |
| CHI,ORPHENESIN | 0.25 |
| DISODIUM EDTA | 0.2 |
| LACTIC ACID | 0.125 |
| O-CYMEN-5-OL | 0.1 |

**TABLE 8**

| Ingredients (INCI Name) | **2955-10** |
|---|---|
| AQUA | to 100% |
| HYDROGENATED POLYDECENE | 12 |
| POLYGLYCERYL-2 DIPOLYHYDROXYSTEARATE | 4 |
| BIS-OCTYLDODECYL DIMER | 3 |
| DILINOLEATE/PROPANEDIOL COPOLYMER | |
| ISOSTEARYL ISOSTEARATE | 3 |
| OCTYLDODECANOL, RIBES NIGRUM SEED OIL, HELIANTHUS ANNUUS SEED OIL UNSAPONIFIABLES, | 2.5 |
| CARDIOSPERMUM HALICACABUM FLOWER/LEAF/VINE | |
| EXTRACT, HELIANTHUS ANNUUS SEED OIL, ROSMARINUS OFFICINALIS LEAF EXTRACT | |
| POLYGLYCERYL-3 DIISOSTEARATE | 2 |
| BETAINE | 2 |
| XYLITOL | 2 |
| TRIHYDROXYSTEARIN | 1 |
| MAGNESIUM STEARATE | 1 |
| MAGNESIUM SULPHATE | 0.7 |
| INULIN, ALPHA-GLUCAN OLIGOSACCHARIDE | 0.5 |
| HYDROXYACETOPHENONE | 0.3 |
| CHLORPHENESIN | 0.25 |
| DISODIUM EDTA | 0.2 |
| LACTIC ACID | 0.175 |
| O-CYMEN-5-OL | 0.1 |

### ADDITIONAL FORMULATIONS

The inventive formulations also are useful for the preparation of products for the care and protection of the skin and mucous membrane, particular in the treatment of skin and mucous diseases such as atopic dermatitis, wound healing, but not limited to these. Additional formulations are given in TABLE 9, TABLE 10, and TABLE 11.

**TABLE 9. DIAPER RASH CREAM**

| **Ingredients (INCI Name)** | **% w/w** |
|---|---|
| AQUA | to 100% |
| ZINC OXIDE | 10 |
| HYDROGENATED POLYDECENE | 5 |
| TRIETHYLHEXANOIN | 5 |
| DIISOPROPYL SEBACATE | 3 |
| POLYGLYCERYL-4 | 3 |
| DIISOSTEARATE/POLYHYDROXYSTEARATE/SEBACATE | |
| BETAINE | 2 |
| XYLITOL | 2 |
| BIS-OCTYLDODECYL DIMER DILINOLEATE/PROPANEDIOL | 1 |
| COPOLYMER | |
| TRIHYDROXYSTEARINE | 1 |
| D-PANTHENOL | 1 |
| PEG-30 DIPOLYHYDROXYSTEARATE | 1 |
| MAGNESIUM SULPHATE | 0.7 |
| DISODIUM EDTA | 0.2 |
| LACTIC ACID | 0.125 |
| PRESERVATIVES | |

**TABLE 10. ACNE PRONE SKIN LOTION**

| **Ingredients (INCI Name)** | **% w/w** |
|---|---|
| AQUA | to 100% |
| C10-18 TRIGLYCERIDES | 5 |
| HYDROGENATED POLYDECENE | 4 |
| GLYCERYL STEARATE AND PEG-100 STEARATE | 4 |
| WATER, TARAKTOGENOS KURZII SEED OIL, NIGELLA SATIVA SEED | |
| OIL, LEPTOSPERMUM SCOPARIUM BRANCH/LEAF OIL, POTASSIUM | 3 |
| LAUROYL WHEAT AMINO ACIDS, PALM GLYCERIDES, CAPRYLOYL | |
| GLYCINE, MAGNOLIA GRANDIFLORA BARK EXTRACT | |
| BETAINE | 2 |
| XYLITOL | 2 |
| SALICYLIC ACID | 1 |
| MICROCRYSTALLINE CELLULOSE AND CELLULOSE GUM | 1.5 |
| ISOSTEARYL ISOSTEARATE | 1 |
| TAPIOCA STARCH | 0.5 |
| TRIHYDROXYSTEARINE | 0.5 |
| BIS-OCTYLDODECYL DIMER DILINOLEATE/PROPANEDIOL | 0.5 |
| COPOLYMER | |
| DISODIUM EDTA | 0.2 |
| XANTHAN GUM | 0.15 |
| LACTIC ACID | 0.05 |
| PRESERVATIVES | |

**TABLE 11. SUN CARE LOTION**

| **Ingredients (INCI Name)** | **% w/w** |
|---|---|
| AQUA | to 100% |
| ZINC OXIDE | 20 |
| DICAPRYLYL CARBONATE | 12 |
| HYDROGENATED POLYDECENE | 5 |
| GLYCERYL STEARATE CITRATE, CETEARYL ALCOHOL, GLYCERYL | 5 |
| CAPRYLATE | |
| ETHYL ALCOHOL | 5 |
| BIS-OCTYLDODECYL DIMER DILINOLEATE/PROPANEDIOL | 2 |
| COPOLYMER | |
| BETAINE | 2 |
| XYLITOL | 2 |
| XANTHAN GUM | 0.5 |
| TRIHYDROXYSTEARINE | 0.5 |
| DISODIUM EDTA | 0.2 |
| LACTIC ACID | 0.2 |
| PRESERVATIVES | |

### RESULTS OF IN VITRO TEST VS VERNIX CASEOSA ON OTHER PROTOTYPES

Results of in vitro testing of these prototypes versus vernix caseosa are reported in TABLE 12:

**TABLE 12**

| **Tested Substances** | **Occlusion Factor (F)** | **Difference vs Vernix** | **WVT R** | **Difference vs Vernix** |
|---|---|---|---|---|
| **Vernix Caseosa** | 23.87 | - | 50.28 | - |
| Petrolatum | 75.65 | + 51.78 | 16.07 | - 34.21 |
| Ref 2954-12 | 23.32 | -0.55 | 50.60 | + 0.32 |
| Ref 2955-10 | 26.28 | + 2.41 | 48.69 | - 1.59 |

These results confirm the synergistic combination of ingredients of the present invention, hydrogenated polydecene, trihydroxystearin, bis-octyldodecyl dimer dilinoleate/propanediol copolymer, betaine and xylitol, formulated in emulsion, and their ability, in a statistically significant way, to resemble the occlusive factor and water vapour transmission rate of vernix caseosa.

### IN VIVO TEST - TEWL REDUCTION CAPACITY

The formulations with occlusion factor and WVTR closest to vernix caseosa (Ref 2952-20, Ref 2953-29; Ref 2954-12; Ref 2955-10) were tested in vivo on human volunteers to evaluate their performance in restoring skin barrier by reducing the TEWL. The investigation was undertaken via non-invasive bioengineering techniques on adult volunteers. The enrolled subjects were adult women and men, Caucasian, with at least 3 of the following conditions:
- Subjects suffering from allergic rhinitis, conjunctivitis and/or asthma.
- Subjects suffering from dermatitis (or that have suffered from dermatitis in early childhood).
- Subjects with very dry skin (xerosis).
- Subjects showing intolerance to textiles (e.g. wool).
- Subjects with a family history of atopy.

The bioengineering technique used to test the product measured transepidermal water loss for the protective barrier effect, by Tewameter TM300 (Courage & Khazaka). The measuring head of the Tewameter TM300 probe is a hollow cylinder that contains two sets of sensors for detecting relative humidity, at different heights. The data obtained is converted into the flux density of the evaporating water, expressed in g/m² hr. TEWL values are registered as baseline values (T₀).

To mimic the compromised condition of impaired skin, irritation and damage of the skin's integrity were experimentally induced by application in occlusion, for 24 hours, of a 2% solution of sodium lauryl sulfate (SLS). Values of TEWL were recorded soon after patch removal (T₁), then the product was applied over the assigned area and values of TEWL were recorded after 1 hour from product application (T_{1_1h}). The product continued to be applied twice a day for 3 consecutive days at intervals of 12 hours on average. TEWL values were recorded after 24, 48, 72 hours (from patch removal) (T₂ T₃ T₄ respectively).

TEWL results obtained are reported in the following tables, recorded as mean value and related standard deviation :

**TABLE 13**

| | T₀ | T₁ | T_{1_1h} | T₂ | T₃ | T₄ |
|---|---|---|---|---|---|---|
| Ref 2952-20 | 7.90 ± 1.05 | 21.51 ± 4.30 | 16.48 ± 4.63 | 21.52 ± 8.38 | 17.30 ± 7.18 | 13.64 ± 5.18 |
| Ref 2953-29 | 8.01 ± 1.11 | 23.98 ± 4.16 | 15.20 ± 4.79 | 22.11 ± 5.05 | 17.98 ± 4.14 | 14.28 ± 3.67 |
| Untreated | 7.32 ± 1.42 | 24.18 ± 8.40 | 23.48 ± 10.68 | 26.32 ± 10.94 | 20.98 ± 6.89 | 19.54 ± 6.96 |

**TABLE 14**

| | T₀ | T₁ | T_{1_1h} | T₂ | T₃ | T₄ |
|---|---|---|---|---|---|---|
| Ref 2954-12 | 8.63 ± 2.26 | 24.14 ± 5.47 | 20.06 ± 4.89 | 23.61 ± 8.32 | 20.51 ± 7.84 | 15.02 ± 3.99 |
| Ref 2955-10 | 8.10 ± 1.98 | 24.15 ± 7.16 | 19.26 ± 5.27 | 23.08 ± 7.42 | 20.62 ± 7.83 | 14.08 ± 3.13 |
| Untreated | 7.85 ± 1.80 | 23.73 ± 5.68 | 23.89 ± 5.72 | 28.20 ± 9.03 | 23.87 ± 5.47 | 19.88 ± 5.01 |

**TABLE 15. Means Variation**

| | T_{1_1h} - T₁ | T₂ - T₁ | T₃ - T₁ | T₄ - T₁ |
|---|---|---|---|---|
| Ref 2952-20 | - 5.06 | -0.02 | -4.24 | -7.90 |
| Ref 2953-29 | -8.78 | -1.87 | - 6.00 | -9.70 |
| Untreated | -0.70 | + 2.14 | - 3.20 | - 4.64 |

**TABLE 16. Means Variation**

| | T_{1_1h} - T₁ | T₂ - T₁ | T₃ - T₁ | T₄ - T₁ |
|---|---|---|---|---|
| Ref 2954-12 | -4.08 | -0.53 | - 3.63 | -9.12 |
| Ref 2955-10 | -4.89 | - 1.07 | - 3.53 | - 10.07 |
| Untreated | + 0.16 | + 4.47 | + 0.14 | - 3.85 |

**TABLE 17. Variation in percentage versus T₁ (damaged skin)**

| | T_{1_1h} - T₁ | T₂ - T₁ | T₃ - T₁ | T₄ - T₁ |
|---|---|---|---|---|
| Ref 2952-20 | - 23.5 | - 0.1 | - 19.7 | - 36.7 |
| Ref 2953-29 | - 36.6 | - 7.8 | -25.0 | - 40.5 |
| Untreated | - 2.9 | + 8.9 | - 13.2 | - 19.2 |

**TABLE 18. Variation in percentage versus T₁ (damaged skin)**

| | T_{1_1h} - T₁ | T₂ - T₁ | T₃ - T₁ | T₄ - T₁ |
|---|---|---|---|---|
| Ref 2954-12 | - 16.9 | -2.2 | - 15.0 | - 37.8 |
| Ref 2955-10 | -20.2 | -4.4 | - 14.6 | -41.7 |
| Untreated | + 0.7 | + 18.8 | + 0.6 | - 16.2 |

**TABLE 19. Statistical comparison among the control times (p value)**

| | T_{1_1h} - T₁ | T₂ - T₁ | T₃ - T₁ | T₄ - T₁ |
|---|---|---|---|---|
| Ref 2952-20 | 0.01 | > 0.05 | > 0.05 | < 0.01 |
| Ref 2953-29 | < 0.001 | > 0.05 | < 0.01 | < 0.001 |
| Untreated | > 0.05 | > 0.05 | > 0.05 | > 0.05 |

**TABLE 20. Statistical comparison among the control times (p value)**

| | T_{1_1h} - T₁ | T₂ - T₁ | T₃ - T₁ | T₄ - T₁ |
|---|---|---|---|---|
| Ref 2954-12 | < 0.01 | > 0.05 | > 0.05 | < 0.0001 |
| Ref 2955-10 | < 0.05 | > 0.05 | > 0.05 | < 0.001 |
| Untreated | > 0.05 | > 0.05 | > 0.05 | > 0.05 |

**TABLE 21. Statistical comparison among the treatment (p value) products versus untreated**

| | T_{1_1h} - T₁ | T₂ - T₁ | T₃ - T₁ | T₄ - T₁ |
|---|---|---|---|---|
| Ref 2952-20 | **< 0.05** | > 0.05 | < 0.01 | **< 0.01** |
| Ref 2953-29 | **< 0.05** | > 0.05 | = 0.001 | = **0.001** |
| Ref 2954-12 | < **0.001** | > 0.05 | > 0.05 | **< 0.05** |
| Ref 2955-10 | **< 0.05** | > 0.05 | < 0.05 | **< 0.01** |

Based on these results, the products show to have a statistically significant effect in reducing the TEWL on damaged skin, from just 1 hour after application and extending through 4 days of applications.

### IN VIVO TEST - SKIN TOLERABILITY TEST

The products were also well tolerated by the skin, based on the in vivo patch test, tested according to the guidelines for human testing in cosmetic science compatibility testing of finished cosmetic products SCCNFP/0068/98. The principle of the test is the application of the test products on the skin of the forearms and the back of adult volunteers. The forearm application evaluates the product's immediate skin irritation potential (ISIP), while the application on the back evaluates the product's irritation potential on the skin (SIP).

The product is kept in contact with the skin for 30 minutes and 48 hours, under occlusive conditions by a specific cell patch (Finn Chambers). Before patch application skin is cleansed of sebum. Clinical observations are carried out by a dermatologist at three control times: 30 minutes after occlusive application to evaluate immediate skin irritation potential, 48 hours after occlusive application and 24 hours after the removal of the occlusion to evaluate the skin irritation potential. The subjects enlisted to test the products were adult women and men, Caucasian, with at least 3 of the following conditions:
- Subjects suffering from allergic rhinitis, conjunctivitis and/or asthma.
- Subjects suffering from dermatitis (or that have suffered from dermatitis in early childhood).
- Subjects with very dry skin (xerosis).
- Subjects showing intolerance to textiles (e.g. wool).
- Subjects with a family history of atopy.

Reactions were evaluated on the following scale:

| | |
|---|---|
| | no erythema |
| +/- | doubtful reaction |
| + | homogeneous pink-red erythema of minimum degree |
| ++ | sharp bright-red erythema, of moderate degree, clear limits, possible faint homogeneous oedema |
| +++ | strong erythema and spread oedema; possible vesicles and/or follicular pustules |

Immediate Skin Irritation Potential and Skin Irritation Potential were separately evaluated. The skin irritation potential of the product, expressed as a percentage of irritation, was evaluated considering the amount and the intensity of the reactions occurring in the total number of the subjects. The following reactions were considered in detail:
positive reactions (from + to +++), observed at occlusive strip removal after 48 hours
positive reactions (from + to +++) observed after 24 hours form the 48h occlusion strip removal
Possible allergic reactions were also considered. They differ from irritative reactions in the following characteristics:
   eczema-like oedema and vesiculation
   reaction is getting to overflow product application area

An allergic reaction that appeared during the test was considered as subjective allergic reaction to one or more of the components of the product.

On the basis of the data reported in literature and of the obtained irritation percentage, the tested product was judged based on this ranking scale

| | |
|---|---|
| 0% < 5% | NON IRRITANT |
| > 5% < 10% | MINIMUM IRRITANT |
| > 10% < 30% | MILD IRRITANT |
| > 30% < 50% | MODERATE IRRITANT |
| > 50% < 80% | STRONG IRRITANT |
| > 80% | MAXIMUM IRRITANT |

Results obtained by Ref 2952-20, 2953-29, 2954-12, 2955-10 were:
0% immediate skin irritation potential
0% skin irritation potential.
No allergic reaction was observed.

Subjects also were asked to give their feedback about the sensation of stinging, itching and burning perceived after the first application of products on the area with induced damaged skin (by application in occlusion of 2% SLS solution). These parameters were scored according to the 4-point scale:
0 = absent,
1 = mild,
2 = moderate,
3 = severe

Results are reported in TABLE 22:

**TABLE 22**

| | **STINGING** | **ITCHING** | **BURNING** |
|---|---|---|---|
| **Ref 2952 Pr 20** | 0 | 0.1 | 0 |
| **Ref 2953 Pr 29** | 0 | 0.1 | 0 |
| **Ref 2954 Pr 12** | 0 | 0 | 0 |
| **Ref 2955 Pr 10** | 0 | 0.1 | 0 |

These results confirm that products applied over damaged skin do not cause a sensation of stinging, itching or burning compared to skin without them.

### MANUFACTURING METHOD

Ref 2952-20, Ref 2953-29, Ref 2954-12, and Ref 2955-10 are water in oil emulsions manufacturing according to standard manufacturing methods. The hydrophilic ingredients and lipophilic ingredients are melted in two separate tanks, then the hydrophilic ingredients are added to lipophilic ingredients under milling (mechanical energy) which continues as the product is cooled down to room temperature.

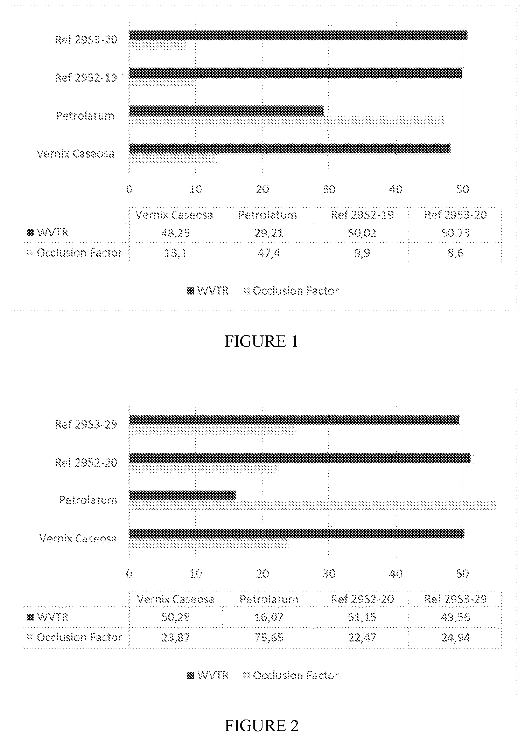

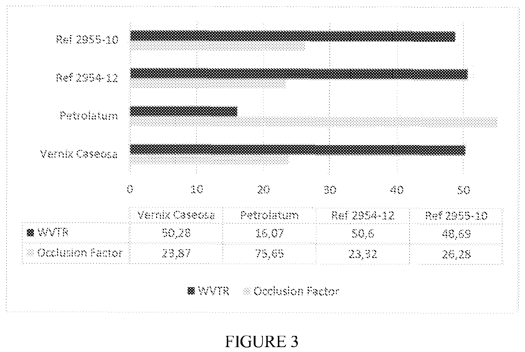

## Claims

1. A topical composition which when applied to the skin or mucosa produces a breathable non-occlusive protective effect, having a water vapour transmission rate and occlusivity similar to that of vernix caseosa, comprising:
a) an isoparaffin with a carbon chain length of 20-60, a hydrogenated oligomer of an alpha olefin, or a combination thereof, the hydrogenated oligomer of the alpha olefin and isoparaffin each having a molecular weight and sufficiently high boiling point to remain on a subject's skin or mucosa after application;
b) a glycerol fatty acid triester;
c) bis-octyldodecyl dimer dilinoleate/propanediol copolymer
d) a sugar alcohol with humectant benefits; and
e) a small amino acid,
wherein the small amino acid is selected from the group consisting of alanine, valine, leucine, isoleucine, glycine, proline, and mixtures thereof, or
wherein the small amino acid is trimethylglycine.

2. The composition of claim 1, wherein the component a) is hydrogenated polyisobutene, hydrogenated polybutene, hydrogenated didecene, hydrogenated tridecene, hydrogenated pentadecene, hydrogenated tetradecene, hydrogenated polydecene, or a mixture thereof,

3. The composition of claim 2, wherein the hydrogenated oligomer of the alpha olefin is hydrogenated polydecene.

4. The composition of claim 1, wherein the glycerol fatty acid triester is glyceryl tristearate, glyceryl tripalmitate, glyceryl benehate, triisostearin, trihydroxystearin, glyceryl triacetyl hydroxystearate, glyceryl tribehenate/isostearte/eicosandioate, glyceryl triacetyl rosinate, or a mixture thereof.

5. The composition of claim 4, wherein the glycerol fatty acid triester is trihydroxystearin.

6. The composition of claim 1, wherein the sugar alcohol is glycerol, erythritol, xylitol, mannitol, sorbitol, inositol, isomalt, maltitiol, lactitol, most preferably xylitol, or a mixture thereof.

7. A topical composition of claim 1, comprising: hydrogenated polydecene, trihydroxystearin, bis-octyldodecyl dimer dilinoleate/propanediol copolymer, trimethylglycine and xylitol.

8. A topical composition according to any of the foregoing claims, for use in treating skin afflicted with a skin disorder selected from atopic dermatitis and wounds.

## Patentansprüche

1. Topische Zusammensetzung, welche bei Anwendung auf der Haut oder Schleimhaut eine atmungsaktive nicht-okklusive Schutzwirkung erzielt, mit einer Wasserdampfdurchlässigkeit und einer Okklusivität ähnlich der von Vernix caseosa, umfassend:
a) ein Isoparaffin mit einer Kohlenstoffkettenlänge von 20 bis 60, ein hydriertes Oligomer eines alpha-Olefins oder eine Kombination davon, wobei das hydrierte Oligomer des alpha-Olefins und Isoparaffin jeweils ein Molekulargewicht und einen ausreichend hohem Siedepunkt aufweisen, um nach der Anwendung auf der Haut oder Schleimhaut eines Individuums zu verbleiben;
b) einen Glycerinfettsäuretriester;
c) Bis-Octyldodecyl-Dimer-Dilinoleat/Propandiol-Copolymer;
d) einen Zuckeralkohol mit Feuchthaltemittelwirkung; und
e) eine kleine Aminosäure,
wobei die kleine Aminosäure ausgewählt ist aus der Gruppe bestehend aus Alanin, Valin, Leucin, Isoleucin, Glycin, Prolin und Gemischen davon, oder
wobei die kleine Aminosäure Trimethylglycin ist.

2. Zusammensetzung nach Anspruch 1, wobei die Kompomente a) hydriertes Polyisobuten, hydriertes Polybuten, hydriertes Didecen, hydriertes Tridecen, hydriertes Pentadecen, hydriertes Tetradecen, hydriertes Polydecen oder ein Gemisch davon ist.

3. Zusammensetzung nach Anspruch 2, wobei das hydrierte Oligomer des alpha-Olefins hydriertes Polydecen ist.

4. Zusammensetzung nach Anspruch 1, wobei der Glycerinfettsäuretriester Glycerintristearat, Glycerintriplamitat, Glycerylbehenat, Triisostearin, Trihydroxystearin, Glycerylacetylhydroxystearat, Glyceryltribehenat/ Isostearat/ Eicosandioat, Glyceryltriacetylrosinat oder ein Gemisch davon ist.

5. Zusammensetzung nach Anspruch 4, wobei der Glycerinfettsäuretriester Trihydroxystearin ist.

6. Zusammensetzung nach Anspruch 1, wobei der Zuckeralkohol Glycerin, Erythrit, Xylit, Mannit, Sorbit, Inosit, Isomalt, Maltit, Lactit, am stärksten bevorzugt Xylit oder ein Gemisch davon ist.

7. Topische Zusammensetzung nach Anspruch 1, umfassend:
hydriertes Polydecin, Trihydroxystearin, Bis-Octyldodecyl-Dimer-Dilinoleat/Propandiol-Copolymer, Trimethylglycin und Xylit.

8. Topische Zusammensetzung nach einem der vorangehenden Ansprüche zur Verwendung bei der Behandlung von Haut, die von einer Hauterkrankung ausgewählt aus atopischer Dermatitis und Wunden betroffen ist.

## Revendications

1. Composition topique qui lorsqu'elle est appliquée sur la peau ou une muqueuse produit un effet protecteur non occlusif respirable, ayant un taux de transmission de vapeur d'eau et une occlusivité similaires à ceux de vernix caseosa, comprenant :
a) une isoparaffine dotée d'une longueur de chaîne de carbones de 20 à 60, un oligomère hydrogéné d'une alphaoléfine, ou une combinaison correspondante, l'oligomère hydrogéné de l'alphaoléfine et l'isoparaffine ayant chacun un poids moléculaire et un point d'ébullition suffisamment élevé pour rester sur la peau ou une muqueuse d'un sujet après application ;
b) un triester d'acide gras de glycérol ;
c) un copolymère de dimère de dilinoléate de bis-octyldodécyle/propanediol
d) un alcool de sucre doté d'avantages d'humectant ; et
e) un petit acide aminé,
le petit acide aminé étant choisi dans le groupe constitué par l'alanine, la valine, la leucine, l'isoleucine, la glycine, la proline et des mélanges correspondants, ou
le petit acide aminé étant la triméthylglycine.

2. Composition selon la revendication 1, le composant a) étant un polyisobutène hydrogéné, un polybutène hydrogéné, le didécène hydrogéné, le tridécène hydrogéné, le pentadécène hydrogéné, le tétradécène hydrogéné, un polydécène hydrogéné, ou un mélange correspondant.

3. Composition selon la revendication 2, l'oligomère hydrogéné de l'alphaoléfine étant un polydécène hydrogéné.

4. Composition selon la revendication 1, le triester d'acide gras de glycérol étant le tristéarate de glycéryle, le tripalmitate de glycéryle, le bénéhate de glycéryle, la triisostéarine, la trihydroxystéarine, le triacétylhydroxystéarate de glycéryle, le tribéhénate/isostéarate/éicosanedioate de glycéryle, le triacétylrosinate de glycéryle ou un mélange correspondant.

5. Composition selon la revendication 4, le triester d'acide gras de glycérol étant la trihydroxystéarine.

6. Composition selon la revendication 1, l'alcool de sucre étant le glycérol, l'érythritol, le xylitol, le mannitol, le sorbitol, l'inositol, l'isomalt, le maltitiol, le lactitol, le plus préférablement le xylitol, ou un mélange correspondant.

7. Composition topique selon la revendication 1, comprenant :
un polydécène hydrogéné, la trihydroxystéarine, un copolymère de dimère de dilinoléate de bis-octyldodécyle/propanediol, la triméthylglycine et le xylitol.

8. Composition topique selon l'une quelconque des revendications précédentes, pour une utilisation dans le traitement d'une peau souffrant d'un trouble de la peau choisi parmi la dermatite atopique et des plaies.
